(19) Europäisches Patentamt
European Patent Office
Office européen des brevets

(11) **EP 2 436 380 B1**

(12) **EUROPEAN PATENT SPECIFICATION**

(45) Date of publication and mention
of the grant of the patent:
**19.11.2014 Bulletin 2014/47**

(51) Int Cl.:
*A61L 15/42* (2006.01)     *A61L 15/58* (2006.01)
*A61L 15/22* (2006.01)     *A61K 9/70* (2006.01)
*C08G 18/48* (2006.01)     *C09J 175/08* (2006.01)

(21) Application number: **10780653.1**

(22) Date of filing: **28.05.2010**

(86) International application number:
**PCT/JP2010/059123**

(87) International publication number:
**WO 2010/137699 (02.12.2010 Gazette 2010/48)**

(54) **PATCH MATERIAL**

FLICKENMATERIAL

PANSEMENT ADHÉSIF

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **29.05.2009  JP 2009131349**

(43) Date of publication of application:
**04.04.2012  Bulletin 2012/14**

(73) Proprietors:
• **Inoac Technical Center Co., Ltd.
Nagoya-shi, Aichi 450-0003 (JP)**
• **Nichiban Co. Ltd.
Tokyo 112-8663 (JP)**

(72) Inventors:
• **SATO, Eisaku
Kanagawa 259-1304 (JP)**

• **SAITO, Ryuji
Kanagawa 259-1304 (JP)**
• **HARAGUCHI, Kazutoshi
Tokyo 112-8663 (JP)**
• **KOIKE, Yoshiko
Tokyo 112-8663 (JP)**
• **IKAI, Tomonori
Tokyo 112-8663 (JP)**

(74) Representative: **Müller-Boré & Partner
Patentanwälte PartG mbB
Friedenheimer Brücke 21
80639 München (DE)**

(56) References cited:
**EP-A1- 0 597 636        WO-A1-00/43433
JP-A- 2 229 876          JP-A- 4 013 618
JP-A- 5 178 741          JP-A- 2002 060 456
JP-A- 2007 031 310       US-A- 5 045 601**

EP 2 436 380 B1

## Description

### TECHNICAL FIELD

The present invention relates to a patch material which is mainly applied to the wound area of the skin.

### BACKGROUND ART

A patch material for a medical use, especially a dressing material is required to prevent water, fungus or virus etc. from invading the body from the outside, and to have flexibility and followability etc. which can follow in the curved surface and motion of the skin. Therefore, thin layer elastomer film having a low elastic modulus like the skin is generally used as a backing material for such patch material. The patch material is also required to have excellent moisture permeability in order to transpire the moisture by sudation through skin outside of the patch material.

Furthermore, if the moisture permeability is low, moisture generated from skin is pooled between the skin and the adhesive layer, adhesion is decreased, the fixing ability of the patch material weakens, and the patch material peels off easily. Moreover a skin disorder occurs easily by increase of resident flora or the like due to storage of water. Therefore, it is also important requirement to give high moisture permeability to the patch material for a medical use.

In order to give high moisture permeability, a patch material for a medical use in which pattern-coating of the adhesive agent was carried out on the moisture permeable film base in the shape of lattice, diamond or the like is suggested. However, there are problems that peripheral part of such patch material peels off easily because such patch material has a section where the adhesive is not applied, in addition, regarding the section where the adhesive is applied, as it has low moisture permeability, the skin get wrinkle and binding of intercellular in the skin is weakened, and thus an amount of peeled corneum is increased.

Accordingly, as a high moisture permeable patch material, a patch material for the skin was also developed using moisture permeable poly (ester urethane) resin film or poly (ether urethane) resin film as a backing material, and acryl adhesive which becomes paste-like liquid at room temperature as an adhesive (Patent Document 1, Patent Document 2).

Patent Document 1: Japanese Laid-Open Patent [Kokai] Publication No. 2003-190205
Patent Document 2: Japanese Laid-Open Patent [Kokai] Publication No. 2005-218496

WO 00/43433 A1 discloses a polyurethane (PU) adhesive having a moisture vapor transmission rate (MVTR) of more than 100g/m2/day comprising the reaction product of :

a) at least one polyether polyol formed from at least one compound selected from the group consisting of ethylene oxide, propylene oxide, 1,2-butylene oxide, 1,4-butylene oxide and mixtures thereof ;
b) at least one low molecular weight polyalkylene glycol having 3 to 7 carbon atoms;
c) at least one crystalline polyester polyol having a melting point of from 40°C to 120°C; and
d) at least one polyfunctional isocyanate;

A wound dressing comprising a backing layer and the PU adhesive is disclosed.

US 5,045,601 A discloses a PU pressure-sensitive adhesive prepared from polyoxyalkylene polyol, isocyanate and HEMA. A dressing comprising the adhesive and a PU backing layer is disclosed, with a MVTR of 6700 g/m2/day for the dressing.

EP 0 597 636 A1 discloses polyurethane pressure-sensitive adhesive, used in wound dressing with a backing layer. The adhesive is prepared from polyisocyanate having a functionality of 2.8 and a mixture of di and trifunctional polyols; and has a MTVR of about 1000 g/m2/day.

### DISCLOSE OF THE INVENTION

### PROBLEMS TO BE SOLVED BY THE INVENTION

However, the patch materials of the above Patent Documents 1 and 2 still have a problem that there is a limit in giving high moisture permeability while maintaining excellent adhesion property.

Therefore, it is an object of the present invention to solve the above mentioned problems in order to provide a patch material having both excellent adhesion property and high moisture permeability.

**MEANS FOR SOLVING THE PROBLEMS**

As a result of conducting extensive studies to solve these problems, the inventors lead to completion of the present invention.

That is to say, the present invention is related to the patch material which comprises a backing material having a water-vapor permeability of 3,000 g/m$^2$·day or more, and an adhesive layer having a water-vapor permeability of 5,000 g/m$^2$·day or more, wherein the adhesive layer comprises a polyurethane adhesive obtained by reacting

(1) an active hydrogen compound having a number average molecular weight of 5,000 or more and an average number of functional groups of 2 or more, and having polyoxyalkylene structure,
(2) an active hydrogen compound having a number average molecular weight of 1,500-5,000 and an average number of functional groups of 1, and having polyoxyalkylene structure, and
(3) an organic polyisocyanate,

wherein an average number of functional groups of all active hydrogen compounds used for obtaining the adhesive is 2-2.6, and a content of ethylene oxide units of all active hydrogen compounds in the adhesive is 3-8% by weight.

In addition, the present invention is related to the above patch material wherein the active hydrogen compound of the above (1) is polyether polyol having an average number of functional groups of 2-3.

Furthermore, the present invention is related to the above patch material showing a water-vapor permeability of 2,000-4,000 g/m$^2$·day, wherein the adhesive layer essentially covers a whole surface of the backing material.

Furthermore, the present invention is related to a polyurethane adhesive having a water-vapor permeability of 5,000 g/m$^2$·day or more, which is obtained by reacting

(1) an active hydrogen compound having a number average molecular weight of 5,000 or more and an average number of functional groups of 2 or more, and having polyoxyalkylene structure,
(2) an active hydrogen compound having a number average molecular weight of 1,500-5,000 and an average number of functional groups of 1, and having polyoxyalkylene structure, and
(3) an organic polyisocyanate,

wherein an average number of functional groups of all active hydrogen compounds used for obtaining the adhesive is 2-2.6, and a content of ethylene oxide units of all active hydrogen compounds in the adhesive is 3-8% by weight.

**EFFECT OF THE INVENTION**

According to the present invention, the patch material having both excellent adhesion property and high moisture permeability can be provided.

**BRIEF DESCRIPTION OF THE DRAWING**

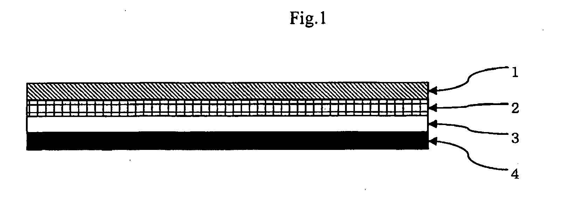

[Fig. 1] is a schematic diagram of a cross-section view of one example of the patch material of the present invention.

**BEST MODE FOR CARRYING OUT THE INVENTION**

For example, the patch material of the present invention can be obtained by providing the above adhesive layer having the specified water-vapor permeability on the above backing material having the specified water-vapor permeability with the common method.

In addition, the patch material of the present invention may further comprise a carrier and/or a release material. The carrier is provided in a releasable form on a surface of the backing material on the side opposite to a surface on which the adhesive layer is provided. The release material is provided in the releasable form on a surface of the adhesive layer on the side opposite to a surface on which the backing material is provided. That is to say, the patch material of the present invention may comprise the carrier, the backing material, the adhesive layer and the release material in this order (Fig. 1). In addition, in the present invention, one or more another layer may be piled between the carrier and the backing material, between the backing material and the adhesive layer and/or between the adhesive layer and the release material. For example, a primer layer, a binder layer or a release agent layer may be provided to enhance the adhesive property or the release property, or a film, non-woven fabrics, woven fabrics or a laminated body thereof may be piled.

The above-mentioned adhesive layer may be provided on the backing material by pattern-coating, e.g. coating in the shape of lattice, diamond or the like, but preferably said adhesive layer essentially covers a whole surface of the backing material to enhance the fixing property to the skin. In addition, in terms of a decrease in the amount of the peeled corneum when the patch material is peeled off from the skin, and a reduction in deterioration of fixability resulting from moisture pooled between the skin and the adhesive layer, preferably the water-vapor permeability of the present invention is 2,000-4,000 g/m$^2$·day.

In the present invention, the measurement of the water-vapor permeability is followed by JIS Z-0208. It is said that the higher water-vapor permeability, the lower stuffy of the backing material or the like. Especially, when the term "high moisture permeability" may be used in the present invention, it shall indicates 2,000 g/m$^2$·day or more in the test followed by JIS Z-0208.

To overcome the above mentioned problems, the backing material of the present invention has the water-vapor permeability of 3,000 g/m$^2$·day or more, preferably 4,000 g/m$^2$·day or more. In addition, upper limit of the water-vapor permeability of the backing material is not limited especially, but generally is about 10,000 g/m$^2$·day or less, preferably about 8,000 g/m$^2$·day or less. The backing material having such water-vapor permeability is easily achieved in non-woven fabrics and knitted cloth, and urethane resin backing material especially useful for a dressing material is known per se (e.g. see Japanese Laid-Open Patent [Kokai] Publication Hei 7-231910 (1995)) and commercially available.

When the backing material is used as a medical tape, preferably it has the water-vapor permeability of 3,000 g/m$^2$·day or more, especially 4,000 g/m$^2$·day or more, and the extensible or inextensible one may be used. It is, e.g., woven-fabrics, non-woven fabrics, knitted cloth, film or the like. It can be selected from polyurethane, polyester, polyvinyl acetate, polyvinylidene chloride, polyethylene, polyethylene telephthalate, aluminum sheet or the like, or a composite material thereof, and they can also be laminated. About a film with low moisture permeability as it is, a porous film containing calcium carbonate or the like, or one which is processed by perforation or the like can be also used. On ground that a low water swellable patch material can be obtained, non-woven fabrics, woven fabrics, knitted cloth or the like are preferred as the backing material.

As the backing material of the present invention, especially as the backing material for a dressing material, the backing material which is produced from urethane resin, e.g. film and the like are preferred. In term of providing flexible and suitable strength, especially enhancing fixing property of the patch material to the skin and reducing uncomfortable feelings during patching, the low water swellable material is preferably used.

In the present invention, in the case that the backing material is produced from urethane resin, it is not restricted especially as long as it has the above mentioned water-vapor permeability, for example, ether urethane resin, ester urethane resin and the like are illustrated. In term of low water swellability, preferably ether urethane resin is selected.

These ether urethane resins having the specified water-vapor permeability are available from BASF or the like. To produce the ether urethane resin, for example, polymerization can be conducted with a conventional method, one-shot method or prepolymer method. In addition, in the case of bulk polymerization with no solvent, polymerization may be conducted in solutions in order to reduce viscosity. Films produced by these polymerization methods include DINTEX FT1080-PE, DINTEX FT1881-PE (UNIPOLYMER), SANPRENE HMP-17A (SANYOU-KASEI) or the like, and each is available.

To the backing material of the present invention, an additive conventionally used, e.g., UV absorbent, aging inhibitor, filler, pigment, colorant, flame retardant, antistatic agent or the like may be added if required. These additives are used in standard amount according to the kind thereof.

The thickness of the backing material of the present invention is preferably 10 $\mu$m or more, especially 15 $\mu$m or more, in terms of enhancing the handleability as the patch material. Furthermore, it is preferably 50 $\mu$m or less, more preferably 40 $\mu$m or less, because of being easy to produce the high moisture permeable backing material and becoming easy to generate the effect of the present invention. In the case of 10 $\mu$m or less, especially 5-10 $\mu$m, the backing material becomes very thin to have difficulty being handled. Therefore, it is required to shape the carrier, e.g., to make the rigidity of the carrier higher than the backing material, or to provide a piece for detaching or the like.

The carrier of the present invention plays a role to reinforce the backing material and improve the manufacturability and usability of the patch material of the present invention. In addition, this carrier is preferably transparent or semitransparent, considering the visibility capable of confirming the patched region when patched. Moreover, it is preferred that this carrier has relatively high elastic modulus to the backing material, and has about 3-20 times the elastic modulus of the backing material. In addition, a surface where the carrier is laminated to the backing material is suitably subjected to various treatments because the carrier is required to be laminated with maintaining suitable adhesiveness to the backing material. Such treatments include a corona treatment, a plasma treatment, a UV treatment, a matting treatment or the like.

If it is difficult for the carrier to be peeled off from the backing material, a slit may be provided near the center of the carrier, or the carriers can be sealed apart so as to be made into two sheets. In addition, a part for detaching may be provided as an holding area, by laminating a tape or film on the upper part of the aparted slit of the carrier. The part for detaching may be film, non-woven fabrics, woven fabrics or laminated body thereof, or may be an adhesive tape, and can be colored. Edge part of the carrier film may be made into waveform or have some cuts, and larger one than the backing material may be used. These are useful to peel off the carrier film and enhance the usability even if the patch material is roll shape.

As the above mentioned carriers, for example, polyolefin such as polyethylene and polypropylene, polyester such as polyethylene telephthalate, polyamide such as nylon, polyvinyl chloride, polyvinylidene chloride or the like may be used. In addition, these are sufficient, not only as a single carrier, but also as a composite carrier laminated with the paper, non-woven fabrics, woven fabrics, knitted cloth or metal foil, and in such carriers, polyolefin and polyester film is preferably used in terms of visibility, cost or the like.

To overcome the above mentioned problems, the adhesive layer of the present invention has the water-vapor permeability of 5,000 g/m$^2$·day or more and preferably 7,000 g/m$^2$·day or more. Upper limit of the water-vapor permeability of the adhesive layer may be 8,000 g/m$^2$·day or less, but the higher the water-vapor permeability, it is more preferred, the upper limit is not limited especially.

In the present invention, adjustment of the water-vapor permeability of the adhesive can be performed with a known method (e.g., see Japanese Laid-Open Patent [Kokai] Publication No. Hei 7-231910 (1995), Japanese Laid-Open Patent [Kokai] Publication No. 2005-58288). In general, high moisture permeability can be obtained by increasing the ethylene oxide (may be abbreviated to EO) unit in the polyurethane adhesive, but if there are too many EO units, when moisture is absorbed, the swelling occurs, and stickiness and reduction of physical property are occurred. In addition, the moisture permeability and adhesiveness can be adjusted by addition of a plasticizer, but insufficiency of inner cohesive force of the polyurethane adhesive is occurred by addition of the plasticizer, and decrease of adhesion is occurred and the adhesive remains on the skin surface when peeled off. Moreover, it is reported that the stimulation by chemical ingredients of these plasticizers can cause a rash and skin inflammation.

The adhesive of the present invention can have both excellent adhesive property and high moisture permeability by adjusting a content of EO units of all active hydrogen compounds in the polyurethane adhesive or the like without addition of the plasticizer or the like. The content of EO units of all hydrogen compounds in the polyurethane adhesive according to the present invention is 3-8% by weight to total amount of the polyurethane adhesive, especially, 4-6% by weight leads to make the gentle-to-skin adhesive having higher moisture permeability and excellent adhesive property.

In addition, even if the adhesive layer of the present invention has the water-vapor permeability of 5,000 g/m$^2$·day or more, it may have an adhesion to Bakelite of 0.5-2.0 N/15mm, preferably 1.0-1.7 N/15mm by the testing method hereinafter, and may have a probe tack of 0.2-1.0 N/5mmφ, preferably 0.5-1.0 N/mmφ by the testing method hereinafter, in the case that thickness of the adhesive layer is 25 μm and thickness of the urethane backing material is 20 μm. Furthermore, adhesion to human skin of the adhesive layer of the present invention is preferably 0.5-1.5 N/15mm, especially around 0.7-1.2 N/15mm after 24 hours of patching. Here, about the adhesion to human skin, a reason why the word "around" is used in the value is for showing that an average of 10 test subjects becomes around the above mentioned value because of a large deviation resulting from individual difference and seasonal variation (e.g., it shows a tendency to be lower in summer, and a tendency to be higher in winter). Adjustment of the adhesion can be performed by the method described herein or conventional methods, the adhesion also can be adjusted by thickness of the adhesive layer and backing material. Namely, when the adhesive layer is thickened, the adhesion tends to be higher, and when it is thinned, the adhesion tends to be lower. When the backing material is thickened, the patch material has higher rigidity, and consequently the adhesion tends to be higher, and when the backing material is thinned, the adhesion tends to be lower. In addition, since it is affected by bending resistance of the backing material per se, it can not generally say, but for example non-woven fabrics or woven fabrics backing material case, the adhesion has the tendency nearly identical to the urethane backing material case.

The polyurethane adhesive of the present invention is preferably flexible by providing relatively long soft segment in a molecular structure, therefore the number-average molecular weight of the active hydrogen compound of the above mentioned (1) is 5,000 or more, preferably 10,000 or more. If the number-average molecular weight is less than 5,000, urethane bonding concentration is raised and adhesion tends to be lower. Upper limit of the number-average molecular weight of the active hydrogen compound of the above mentioned (1) is not limited especially, but 15,000 or less, especially 12,000 or less of the number-average molecular weight is preferred in terms of increasing the adhesion of the obtained adhesive, and decreasing the adhesive residue (the adhesive remains on the skin when the patch is peeled off from the skin).

To secure better adhesive property (e.g., hardness, tack feeling, adhesion or the like with the finger-tack sensory evaluation), more preferably, the number-average molecular weight of the active hydrogen compound of the above mentioned (1) is 5,000-10,000.

In addition, the average number of functional groups (F) of the active hydrogen compound of the above mentioned (1) is not particularly limited insofar as it is 2 or more, but it is preferably 2-3. Since defect such as adhesive residue decreases by enhancement of cohesive force, the average number of functional groups is preferably 2 or more. On the other hand, since the cohesive force does not become strong too much and the adhesion may be raised, and gelatification in producing the adhesive is suppressed, the average number of functional group is preferably 3 or less.

By changing blend ratio of the active hydrogen compounds with F=2 and F=3 of the above mentioned (1), F theoretically can be set freely between 2 and 3.

As the active hydrogen compound of the above mentioned (1) of the present invention, polyether polyol; poly(ether-ester) polyol obtained by partly esterification of polyether polyol, and polyalkylene (e.g., ethylene and propylene etc.) oxide diamine having amino group and the like are exemplified, and polyether polyol is preferred. As the polyether polyol, e.g., as those having the average number of functional groups of 2, polyether glycol such as polyethylene glycol, polypropylene glycol and polytetramethylene ether glycol, which are obtained by ring-opening polymerization of ethylene oxide, propylene oxide, tetrahydrofuran or the like, and polyether glycol obtained by copolymerization thereof; as those having the average number of functional groups of 3, polyether polyol such as polyethylene polyol, polypropylene polyol, polytetramethylene ether polyol and the like, which are obtained by ring-opening polymerization of ethylene oxide, propylene oxide, tetrahydrofuran or the like using an initiator having 3 or more active hydrogen residues such as glycerin, trimethylol propane, pentaerythritol, sorbitol, sucrose or the like; or the mixture of these 2 or more polyols having the average number of functional groups of 2 or 3 or more is preferred. Concretely, SANNIX series of SANYOU-KASEI, Actcol series of MITSUI-KAGAKU-POLYURETHANE, ADEKA polyether series of ASAHI-DENKA, Acclaim series of Lyondell or the like can be used.

In the present invention, the active hydrogen compound of the above mentioned (1) may be used alone, and the mixture of 2 or more compounds which are different, for example, in the number-average molecular weight, the average number of functional groups, the kind of monomer units or the like may also be used.

On ground that the high water-vapor permeability is obtained without reducing of adhesion, the polyurethane adhesive of the present invention is manufactured with the active hydrogen compound of the above mentioned (2) having the number-average molecular weight of 1,500-5,000 and the average number of functional groups of 1, and having a polyoxyalkylene structure. The number-average molecular weight of this active hydrogen compound of the above mentioned (2) is 1,500-5,000, preferably 2,000-5,000.

In particular, the polyurethane adhesive of the present invention is preferably obtained by combining the active hydrogen compound of the above mentioned (1) having the number-average molecular weight of 5,000 or more, especially of 5,000-12,000, with the active hydrogen compound of the above mentioned (2), preferably with the active hydrogen compound of the above mentioned (2) having relatively high molecular weight, e.g. 2,000-5,000, because the adhesion and the water-vapor permeability are enhanced. The polyurethane adhesive obtained by such way is advantageous in terms of the suppression of stickiness or the like which arises by using the plasticizer, because the water-vapor permeability thereof can be enhanced without the plasticizers.

As the active hydrogen compound of the above mentioned (2), polyoxyalkylene monool and polyalkylene (e.g., ethylene, etc.) oxide monoamine or the like are exemplified, and polyoxyalkylene monool is preferred. As the polyoxyalkylene monool, for example, alkyl poly(oxyethylene/oxypropylene) monool and alkyl polyoxytetramethylene monool, which are obtained by chain-opening polymerization of ethylene oxide, propylene oxide, tetrahydrofuran or the like with alkyl monool as an initiator, and the mixture of these 2 or more ones are exemplified.

In the present invention, the active hydrogen compound of the above mentioned (2) may be used alone, and the mixture of 2 or more compounds which are different, for example, in the number-average molecular weight, the kind of the monomer units or the like may also be used.

The amount of the active hydrogen compound of the above mentioned (2) used in the manufacture of the polyurethane adhesive of the present invention is not limited especially, but 5-55 weight parts, more preferably 20-40 weight parts are preferably used based on 100 weight parts of the active hydrogen compound of the above mentioned (1), on ground that the excellent adhesion and water-vapor permeability can be obtained.

To obtain excellent adhesive property, the active hydrogen compound used in the manufacture of the polyurethane adhesive of the present invention is preferably consisted of only active hydrogen compounds of the above mentioned (1) and (2), but can contain other active hydrogen compound such as monool and polyol in the range which does not produce a problem in the adhesive property. As such active hydrogen compounds such as monool and polyol, general compounds such as acryl monool, ester polyol, polycarbonate polyol and the like can be used.

The average number of functional groups of all active hydrogen compounds used to obtain the polyurethane adhesive of the present invention is the sum of the products of an average number of functional groups of each active hydrogen compound and percentage of added weight thereof, about all active hydrogen compounds which are reacted with the organic polyisocyanate of the above mentioned (3), including the active hydrogen compounds of the above mentioned (1) and (2). On ground that the excellent adhesive property can be obtained, this average number of functional group is 2-2.6, preferably 2.05-2.4.

It is checked that the alkylene oxide which is a component of the polyurethane adhesive of the present invention, that is, the active hydrogen compounds of the above mentioned (1) and (2) having a polyoxyalkylene structure usually has high safety to the human body. Similarly, the polyurethane, which has a segment of them, also has high safety, and it is actually used as a macromolecule material for a medical use. In addition, by selection of kind of the active hydrogen compound of above mentioned (1) and (2), the balance of hydrophilicity and hydrophobicity of the segment can be adjusted so as to result in the adhesive which has high transparency, good adhesion to skin, flexible, high conformability and low irritancy, with the help of flexibility of molecule by urethane bonding. Therefore, it is suitable for the use of patching to the human body, and suitable as the adhesive for a medical patch material. In this case, it can be a novel adhesive which satisfies all of adhesiveness, safety, stability as the material, cost performance and the like.

As the organic polyisocyanate of the above mentioned (3) used in the polyurethane adhesive of the present invention, all organic polyisocyanate which can be used generally in the manufacture of polyurethane resin can be used. Concretely, aromatic diisocyanate such as 2,4-trilene diisocyanate, 2,6-trilene diisocyanate, 2,2'-diphenylmethane diisocyanate, 4,4'-diphenylmethane diisocyanate, 2,4'-diphenylmethane diisocyanate or the like; aliphatic diisocyanate such as tetramethylene diisocyanate, hexamethylene diisocyanate, lysyin diisocyanate or the like; alicyclic diisocyanate such as isophoron diisocyanate, hydrogenated trilene diisocyanate, hydrogenated diphenylmethane diisocyanate or the like; and further organic diisocyanate such as the mixture thereof are preferred, but polyisocyanate obtained by the partially modification thereof with urethanation, urethodionation, carbodiimidation or the like can also be used. Furthermore, isocyanate prepolymer which is prepolymerized with polyol can also be used.

The NCO/OH molar ratio of the active hydrogen compound and the organic polyisocyanate is preferably 0.7/1.00-1.00/1.00 because of suppression of gelatification in the reaction and enhancement of the adhesion. If the residual NCO amount is low, temporal stability improve, and consequently the ratio of 1.05/1.00 or less, especially 1.00/1.00 or less is preferred. In addition, the ratio of 0.7/1.00 or more is preferred because potential of bleed-out is reduced by the reduction of the amount of the residual active hydrogen compound.

In the production of the polyurethane adhesive of the present invention, for example, the conventional methods such as a bulk polymerization (solid reaction) method wherein the reaction is performed in the melted state, a solution polymerization method or the like can be used. A solvent used in the solution polymerization method concretely includes ketone solvent such as methyl ethyl ketone, methyl isobutyl ketone, cyclohexanone or the like, ester solvent such as ethyl acetate, butyl acetate or the like, ether solvent such as dioxane, tetrahydrofuran or the like, glycol ether solvent such as cellosolve, carbitol or the like, glycol ether acetate solvent such as cellosolve acetate, amide solvent such as dimethyl acetamide, dimethyl formamide or the like, aromatic hydrocarbon solvent such as toluene, xylene or the like, and alcohol solvent such as methanol, ethanol, isopropanol or the like, and further mixed solvents thereof.

In the production of the polyurethane adhesive of the present invention, a catalyst, an additive or the like can be used if required. The catalysts include conventional urethanation catalysts including nitrogen-containing compounds such as triethylamine, triethylenediamine and the like, and organometallic compounds such as dibutyltin dilaurate, tin octylate, tin stearate and the like. The additives include, for example, UV absorbers such as substituted benzotriazoles and the like, anti-oxidants such as phenol derivatives and the like, anti-hydrolyzing agents, and the like.

Moreover, in the production of the polyurethane adhesive as above, a chain extender can be used in order to increase the cohesive force and to avoid the problem of the residual adhesive or the like. A total of the number of moles of the chain extenders used is preferably less than or equal to the total of the number of moles of the active hydrogen compounds in the whole system, especially less than or equal to 1/2 thereof. In the case that the total of the number of moles of the chain extenders used is less than or equal to the total of the number of moles of the active hydrogen compounds in the whole system, especially less than or equal to 1/2 thereof, mechanical characteristic become worse by reducing the amount of the urethane bonding which has strong cohesive force, but it is preferred because its adhesion is enhanced.

The adhesive property of the polyurethane adhesive can be changed in combination with a polyisocyanate hardener. An increase of cohesive force in combination with the polyisocyanate hardener is useful to improve the defects such as residual adhesive or the like. Moreover, physical property of the final adhesive can be adjusted finely by changing of the additive amount of this polyisocyanate hardener. For example, in order to increase cohesive force of adhesive, the additive amount is increased, and in order to increase tack of the adhesive, the additive amount is reduced. As the polyisocyanate used as the hardener, the organic polyisocyanate of the above mentioned (3) can also be used, but polyol adduct of isocyanate obtained by the reaction of them and polyol having 2 or more functional groups is preferred, and polymeric polyisocyanate, denaturated isocyanurate and denaturated carbodiimide are also preferred. Concretely, it includes CORONATE L, CORONATE HL, CORONATE 3041, CORONATE 2030, CORONATE 2031, CORONATE HX, MILLIONATE MTL, MILLIONATE MR and the like manufactured by NIPPON POLYURETHANE INDUSTRY Co. Ltd. Its usage is preferably 0.5-5 weight parts to 100 weight parts of the above mentioned polyurethane adhesive (both as a solid content), especially, in the case of CORONATE L, 1-3 weight parts (both as a solid content) are preferred.

The adhesive layer of the patch material of the present invention may comprise the adhesive other than polyurethane adhesive in the range which does not spoil the effect of the present invention. However, since the adhesive layer of the present invention may not comprise the acryl adhesive, according to the present invention, the patch material which almost has neither smell particular to the acryl adhesive nor worries about skin irritation or the like can be obtained.

The thickness of the adhesive layer of the patch material of the present invention is not limited especially, but preferably 10 μm or more in terms of ensuring fixing property to the skin and the balance with the thickness of the backing material, and preferably 40 μm or less on ground that the water-vapor permeability is reduced when the adhesive layer becomes too thick.

In addition, in the usage of covering the skin, in the case that the thickness of the adhesive layer of the patch material is around 3-7 $\mu$m, the effects such as the followability to the skin as the patch material and inconspicuousness or the like can be given to the patch material.

As the release material of the present invention, the conventional material in the field of a patch material can be used. For example, paper base materials such as wood free paper, glassine paper or the like which are subjected to a silicone mould release treatment, polyester film or the like can be used. In addition, fabric weight of the release material is not limited but in general, preferably about 50-150 g/m$^2$, more preferably about 60-100 g/m$^2$. 1 or 2 or more linear release liner division parts are provided around the central part of the release material so as to divide the outline thereof, thereby it comes to be able to perform patching work without touching an adhesive face because one release material remains even if another one is removed, and consequently the workability improves. In the case that the patch material is in the form of a roll, it is effective especially for being easy to peel off the release material, and for improving usability. In addition, in order to facilitate peeling off 2 or more release materials from adhesive, placing the release material hunched over other one, or folded, is effective to improve usability.

In the patch material of the present invention, pad can be used. As the pad, gauze, and non-woven fabrics of rayon, polyethylene, polyester or polypropylene, and the like, which have fabric weight of around 2-100 g/m$^2$, can be used, and suitably, it can be placed on the center of the surface to which the adhesive is applied.

The numerical range shown by the upper limit and the lower limit disclosed herein may be excluded partly by being optionally narrowed (alternatively, one point or some points in the range may be excluded), even in the range after the exclusion, same effect is generated before the exclusion.

EXAMPLES

The following provides a more detailed explanation in the present invention by listing examples thereof, but the present invention is not limited to these examples. In the following synthetic examples, examples and comparative examples, in principle, "part(s)" and "%" mean "part by mass" and "% by mass" respectively.

1. Preparation of the polyurethane adhesive

In the reaction vessel equipped with thermometer, stirrer and condenser, as shown in the Table 1, to the designated amount of polyether polyol A (copolymer of EO units and propylene oxide (PO) units using glycerin as an initiator, number-average molecular weight 10,000, F = 3), polyether polyol B (copolymer of EO units and PO units, number-average molecular weight 4,000, F = 2), polyether polyol C (polymer of PO units, number-average molecular weight 10,000, F = 2), polyether monool D (polymer of PO units using methanol as an initiator, number-average molecular weight 3,500, F = 1), polyether polyol E (polymer of PO units, number-average molecular weight 3,000, F = 2), polyethylene glycol A (number-average molecular weight 11,000, F = 2), acryl monool A (number-average molecular weight 1,400, F = 1), polyether polyol F (polymer of PO units using glycerin as an initiator, number-average molecular weight 10,000, F = 3), or polyether monool G (polymer of PO units using methanol as an initiator, number-average molecular weight 5,000, F = 1), toluene and ethyl acetate were added and adjusted so that the solid content was 50%, then 0.02% to the above solid content of dibutyltin dilaurate as a urethanation catalyst was added and mixed at 40°C, then hexamethylene diisocyanate prepolymer (Mw 600, F = 2) as an organic diisocyanate was added so that molar ratio became 90 of isocyanate groups in the isocyanate prepolymer against 100 of hydroxyl groups in the active hydrogen compound, and was reacted at 80°C. The reaction generated heat and the internal temperature became 80°C, and viscosity also rose with time. It was reacted for 7 hours maintaining the temperature at 80°C while being diluted by properly adding ethyl acetate, to yield a polyurethane adhesive (A)-(J) as a homogeneous transparent solution. Their final solid content and viscosity was shown in Table 1.

Constructing material of these polyurethane adhesive (A)-(J) and their ratio and physically properties are shown in Table 1.

[Table 1]

Table 1

| Active hydrogen compound | Average number of functional groups | Number-average molecular weight | Content of EO units (wt%) | Polyurethane adhesive | | | | | | | | | |
|---|---|---|---|---|---|---|---|---|---|---|---|---|---|
| | | | | A | B | C | D | E | F | G | H | I | J |
| Polyether polyol A | 3 | 10,000 | 12 | 43 | 43 | 43 | 43 | 85 | 30 | 25 | 43 | 43 | 43 |
| Polyether polyol B | 2 | 4,000 | 24 | — | — | — | — | — | 30 | — | — | — | — |
| Polyether polyol C | 2 | 10,000 | 0 | 21 | 33 | 22 | — | — | — | 21 | 18 | 9 | 8 |
| Polyether polyol D | 1 | 3,500 | 0 | 36 | 24 | — | 35 | — | 40 | 36 | 36 | — | 36 |
| Polyether polyol E | 2 | 3,000 | 0 | — | — | 35 | — | — | — | — | — | — | — |
| Polyethylene glycol A | 2 | 11,000 | 100 | — | — | — | 22 | — | — | — | 3 | — | 13 |
| Acryl monool A | 1 | 1,400 | 0 | — | — | — | — | 15 | — | — | — | — | — |
| Polyether polyol F | 3 | 10,000 | 0 | — | — | — | — | — | — | 18 | — | — | — |
| Polyether monool G | 1 | 5,000 | 0 | — | — | — | — | — | — | — | — | 48 | — |
| Content of EO units in all active hydrogen compounds (wt%) | | | | 5.2 | 5.2 | 5.2 | 27.2 | 10.2 | 10.8 | 3 | 8.2 | 5.2 | 18.2 |
| Average number of functional groups in all active hydrogen compounds | | | | 2.07 | 2.19 | 2.43 | 2.08 | 2.70 | 1.90 | 2.07 | 2.07 | 1.95 | 2.07 |
| Solid content concentration in the adhesive (%) | | | | 45 | 50 | 50 | 52.2 | 60 | 42 | 45.5 | 48 | 25.7 | 25.7 |
| Viscosity of the adhesive (CP) | | | | 3700 | 4230 | 5952 | 6276 | 3000 | 1820 | 710 | $>10^5$ | 280 | 382 |

EP 2 436 380 B1

2. Preparation of the patch material

(Example 1)

100 parts of the above prepared polyurethane adhesive (A) and 2 parts of the COLONATE L (manufactured by NIPPON POLYURETHANE INDUSTRY Co. Ltd.) was mixed homogeneously and then degassed, and applied on the polyester film which underwent a release treatment (release material, thickness was 50 $\mu$m) with a knife coater so that thickness of dry film was 25 $\mu$m, then dried hard for 3 minutes at 120°C. To the obtained adhesive layer, laminated material of a film base of the ether urethane resin having 20 $\mu$m thickness (backing material, water-vapor permeability 3,300 g/m$^2$·day) and a polyester film having 40 $\mu$m thickness (carrier) was affixed so that the surface of the film base was faced to the adhesive layer, after that, preserved 5 days under atmosphere of 50°C in the hot air dryer to finish the bridging reaction of the adhesive layer, and prepared the patch material.

(Example 2)

The patch material was prepared in the same manner as in Example 1 except for using the above prepared (B) as a polyurethane adhesive instead of (A), and measured and evaluated.

(Example 3)

The patch material was prepared in the same manner as in Example 1 except for using the above prepared (G) as a polyurethane adhesive instead of (A), and measured and evaluated.

(Example 4)

The solution of polyether polyurethane elastomer (LUCKSKIN US2268 manufactured by SEIKOH CHEMICALS Co. Ltd.) was applied on the silicone-treated surface of a polyester film with 75 $\mu$m thickness of which one side was treated with silicone so that the thickness after drying was 5 $\mu$m, and dried to obtain the backing material comprising polyurethane film (water-vapor permeability 5,150 g/m$^2$·day). On the polyether polyurethane elastomer layer of this backing material, degassed homogenous mixture of 100 parts of the above prepared polyurethane adhesive (A) and 2 parts of COLONATE L (manufactured by NIPPON POLYURETHANE INDUSTRY Co. Ltd.) was applied with a knife coater so that the thickness of said adhesive after drying was 5 $\mu$m, and then dried hard at 120°C for 3 minutes. To the obtained adhesive layer, another polyester film with 75 $\mu$m thickness of which one side was treated with silicone was affixed so that the silicone-treated surface was faced to the adhesive layer, and then cut to prepare the patch material, and measurement and evaluation were performed.

(Example 5-8)

In each example, the patch materials were prepared in the same manner as in Example 4, and measured and evaluated. However, the application was performed so that the thickness of the adhesive after drying were 10 $\mu$m in Example 5, 15 $\mu$m in Example 6, 20 $\mu$m in Example 7, 25 $\mu$m in Example 8.

(Comparative Example 1)

The patch material was prepared in the same manner as in Example 1 except for using the above prepared (C) as a polyurethane adhesive instead of (A), and measured and evaluated.

(Comparative Example 2)

The patch material was prepared in the same manner as in Example 1 except for using the above prepared (D) as a polyurethane adhesive instead of (A), and measured and evaluated.

(Comparative Example 3)

The patch material was prepared in the same manner as in Example 1 except for using the above prepared (E) as a polyurethane adhesive instead of (A), and measured and evaluated.

(Comparative Example 4)

The patch material was prepared in the same manner as in Example 1 except for using the above prepared (F) as a polyurethane adhesive instead of (A), and measured and evaluated.

(Comparative Example 5)

The patch material was prepared in the same manner as in Example 1 except for using the above prepared (H) as a polyurethane adhesive instead of (A), and measured and evaluated.

(Comparative Example 6)

The patch material was prepared in the same manner as in Example 1 except for using the above prepared (I) as a polyurethane adhesive instead of (A), and measured and evaluated.

(Comparative Example 7)

The patch material was prepared in the same manner as in Example 1 except for using the above prepared (J) as a polyurethane adhesive instead of (A), and measured and evaluated.

(Comparative Example 8)

To the backing material comprising polyurethane film prepared in Example 4, acryl adhesive (a solution of 2-ethylhexcyl acrylate/vinyl acetate/acrylic acid = 85/11/4 wt% in ethyl acetate) was applied so that the thickness after drying was 5 $\mu$m, and then to the obtained adhesive layer, polyester film with 75 $\mu$m thickness of which one side was treated with silicone was affixed so that the silicone-treated surface was faced to the adhesive layer, and then cut to prepare the patch material, and the measurement and evaluation were performed.

(Commercial Product a)

As a commercially available adhesive tape for a medical use, OPSITE IV-3000 (manufactured by Smith & Nephew, No.4973, Lot0510, Plant No 9285) was used.

3. Evaluation of property and organoleptic property of the patch material

About the patch materials obtained above in Examples 1-3 and Comparative Examples 1-7, adhesion to Bakelite, probe tack, holding force, water-vapor permeability and finger tack (organoleptic property) were evaluated according to the method hereinafter. The results were shown in Table 2. The test piece is prepared by cutting the patch material to 15 mm width and 60 mm length so that the direction of roll flow was long side (direction of MD) unless otherwise described especially. In addition, measurement was performed in the state that the carrier and the release material were peeled off from the patch material.

(1) Adhesion to Bakelite

According to JIS Z-0237, under atmosphere at 25°C, the test piece having 15 mm width was affixed to Bakelite panel (phenol resin plate, manufactured by SUMITOMO BAKELITE, PL-1102), pressed back and forth with 2 kg rubber roll in the rate of 300 mm/min, after staying at 20 minutes, peel force was measured in peeling angle 90° or 180°, and peeling rate 300 mm/min.

(2) Probe tack

According to ASTM D-2979, under atmosphere at 25°C, using NS probe tack tester (manufactured by NICHIBAN), measurement was performed under the condition of diameter of pillar contactor (probe): 5 mm, pressure 0.98 N/cm$^2$, contact time; 1 second, peeling rate; 10 mm/sec.

(3) Holding force

To the patch material covered with carton tape, according to JIS Z-0237, under atmosphere at 25°C, double-sided adhesive tape (for industry) (manufactured by NICHIBAN) was affixed, and it was affixed to a glass plate on which Ippanyupo FPG 130 (manufactured by YUPO CORPORATION) was pasted, so that the area was 12 mm (horizontal) x 20 mm (vertical), and pressed back and forth with 2 kg rubber roll in the rate of 300 mm/min. After staying 20 minute, the patch material was hanged down perpendicularly and 200 g load was added and slid length (mm) after 30 minute or 60 minute was measured.

(4) Water-vapor permeability

The water-vapor permeability was evaluated as an amount of moisture which penetrates the test piece per unit area in a definite period of time. Concretely, under atmosphere at 40°C, when the relative humidity in one of the space which was partitioned with the test piece was 90%, and another of the space maintained a dry condition with desiccant, an amount of moisture (g) which penetrate the test piece for 24 hours was measured, and it was converted into per 1 $m^2$ of the test material. The measurement was carried out according to JIS Z-0208. A cup containing about 16 g of calcium chloride desiccant was covered with a circular test piece of which diameter was about 10 mm larger than the internal diameter of the cup, and further was covered with rubber packing and ring and screwed shut so as not to displace the test piece. In the case of measurement of the water-vapor permeability of the adhesive layer, the test piece was prepared by supporting the adhesive layer to be measured with a large-mesh nylon net which did not affect the water-vapor permeability of the adhesive layer, and measured in the same manner as above. After measurement of the total weight of this test piece, it was placed in the constant temperature and humidity vessel under atmosphere of 40°C and 90%RH, the change of weight per definite period of time was measured, and the water-vapor permeability was evaluated according to a following formula:

$$\text{Water-vapor permeability (g/m}^2\cdot\text{day)} = W \times 24000/S$$

[Wherein S refers to moisture permeation area ($cm^2$), W refers to mass increase per 1 hour (g/hr).]

(5) Finger tack (sensory evaluation)

After the polyester film was peeled off from the patch material, the adhesive layer was touched directly by thumb, and the sensory evaluation of the adhesive (hardness, tack feeling, and adhesion) was performed according to following.

a) Hardness

The patch material was picked so that forefinger contacts to the backing material side and thumb contacts to the adhesive side, and when the thumb was pushed down to and peeled off from the adhesive, the hardness of the adhesive felt at the finger tip was evaluated according to the following criteria.

Excellent: When the thumb is pushed down, there is moderate hardness of the adhesive.
Good: When the thumb is pushed down, the adhesive is somewhat hard or somewhat soft.
Suitable: When the thumb is pushed down, the adhesive is hard or soft.

b) Tack feeling

The tack feeling was evaluated according to the following criteria with the same operation in the above evaluation for hardness. "Quickly" means peeling time of 0.5 second or less which can be attained easily.

Excellent: When the thumb is peeled off from the adhesive quickly, suitable resistance is felt.
Good: When the thumb is peeled off from the adhesive quickly, somewhat strong or somewhat weak resistance is felt.
Suitable: When the thumb is peeled off from the adhesive quickly, strong or weak resistance is felt.

c) Adhesion

The adhesion was evaluated according to the following criteria with the same operation in the above evaluation for hardness. "Slowly" means the time around 1-2 second.

Excellent: When the thumb is peeled off from the adhesive slowly, suitable resistance is felt.
Good: When the thumb is peeled off from the adhesive slowly, somewhat strong or somewhat weak resistance is felt.
Suitable: When the thumb is peeled off from the adhesive slowly, strong or weak resistance is felt.

[Table 2]

Table 2

| Entry | | Patch material | | | | | | | | | |
|---|---|---|---|---|---|---|---|---|---|---|---|
| | | Example 1 | Example 2 | Comparative Example 1 | Comparative Example 2 | Comparative Example 3 | Comparative Example 4 | Example 3 | Comparative Example 5 | Comparative Example 6 | Comparative Example 7 |
| Polyurethane adhesive | | A | B | C | D | E | F | G | H | I | J |
| Cross-linker COLONATE L | | 2 | 2 | 2 | 2 | 2 | 2 | 2 | 2 | 2 | 2 |
| Water-vapor permeability of adhesive layer (g/m$^2$·day) | | 7310 | 5490 | 5840 | 8990 | 8020 | 7370 | 5531 | 5720 | 6375 | 7882 |
| Properties of the patch material | Adhesion to Bakelite [N/15mm] | 1.33 | 1.26 | 1.71 | 1.14 | 1.04 | 1.81 | 1.61 | 1.24[1] | 1.41 | 1.08[1] |
| | Probe tack [N/5mmφ] | 0.57 | 0.5 | 0.4 | 0.33 | 0.41 | 0.63 | 0.91 | 0.7 | 0.51 | 0.5 |
| | Holding force [mm] | 0.3 | 0.2 | 0.1 | 0.1 | 0.1 | 2.5 | 0.4 | 0.2 | 0.1 | 0.1 |
| | Water-vapor permeability [g/m$^2$·day] | 2556 | 2687 | 2671 | 3102 | 2710 | 2860 | 2689 | 2899 | 2932 | 3058 |
| | Finger tack [organoleptic property] — Hardness | excellent | excellent | good | excellent | suitable | suitable | excellent | good | good | good |
| | Finger tack [organoleptic property] — Tack feeling | excellent | excellent | suitable | good | suitable | suitable | excellent | good | suitable | suitable |
| | Finger tack [organoleptic property] — Adhesion | excellent | excellent | good | good | suitable | suitable | excellent | good | suitable | suitable |

1) anchorage failure on whole surface

Adhesion to Bakelite: measured in 180° of peeling angle

Holding force: expressed in the slid length after 60 minutes

EP 2 436 380 B1

4. Practical evaluation of the patch material

About Example 1 and "Commercially Product a", (6) adhesion to skin, (7) condition of adhesion, (8) pain with peeling off, (9) residue of adhesive, (10) index of skin irritation, (11) amount of peeled corneum and (12) re-patch property to skin, were evaluated according to the method hereinafter, and the average was calculated. The result was shown in Table 3. In the following evaluation, the test subjects were 10 adults, and the test piece was the patch material which was cut into 15 mm width and 60 mm length so that the direction of roll flow was long side (direction of MD) unless otherwise indicated. Carrier and release material was peeled off from the patch material, and then measurement was performed.

(6) Adhesion to skin

The test subjects entered to the measurement room under condition of 25°C and 50%RH before 20 minutes of patching, release material was peeled off from the test piece and two test pieces were patched to a forearm inner side part at a transverse direction per one test subject, and then the carrier was peeled off. After 6 and 24 hours of patching, it was peeled off with Instron-type tensile tester in the peeling angle 90° and in the tension rate of 100 mm/min, and adhesion to skin was measured.

(7) Condition of adhesion

In the (6), before measurement of the adhesion to skin after 24 hours of patching, the condition of adhesion to skin of the test piece was observed and scored according to the following criteria.
5: the whole surface is adhered, 4: peripheral part of the patch material is peeled off, 3: around 25% is peeled off, 2: around 50% is peeled off, 1: drop off

(8) Pain with peeling off

After 24 hours of patching, when measuring of the adhesion to skin in the (6), the pain with peeling off was also scored according to the following criteria.
4: Almost nothing, 3: slightly painful but no problem, 2: Painful and be a problem, 1: Very painful and be a problem

(9) Residue of adhesive

After 24 hours of patching, after measuring of the adhesion to skin in the (6), the residual property of the adhesive on the skin (residual adhesive) was scored according to the following criteria.
4: Almost nothing, 3: Those with some, 2: There is somewhat on whole surface, 1: There is much on whole surface

(10) Index of skin irritation

On the skin reaction where the test piece was patched, the test piece was peeled off after 24 hours of patching, and skin irritation of the part where the test piece was patched was evaluated according to Japanese Criteria after 1 hour and 24 hours of peeling.
Here, "Japanese Criteria" is provided by Patch Test Studying Group in Japan. Concretely, following -, ±, +, ++, +++ and ++++ were scored as 0, 0.5, 1, 2, 3 and 4 respectively, and according to the following formula, average of the evaluation results of each test subject was increased 100 times to evaluate the index of skin irritation.

Criteria

- : no reaction
± : mild erythema
+ : erythema
++ : erythema + edema
+++ : erythema + edema + papule
++++ : erythema + edema + papule, serosity papule, phlyctenule

$$\text{Index of skin irritation} = (\text{Sum of scores}/\text{number of test subject}) \times 100$$

On the index of skin irritation, around 10 means a little irritation, around 30 means a stronger irritation, and 50 or more means a large irritation.

(11) Amount of peeled corneum

The test pieces were patched to the test subject for 24 hours, and then were peeled off, and these test pieces were dyed by immersing to the following stain solution for 24 hours, and washed with distilled water. The amount of corneum which was transferred to the adhesive surface of the test piece after peeling was observed with optical microscope, and the amount of peeled keratinocyte was measured by area (%) which accounts for the whole. In the case that the amount of peeled corneum is 100%, it means that the keratinocyte was adhered to whole adhesive surface.

Composition of stain solution:

| | |
|---|---|
| Gentian Violet | 1.0% |
| Brillian Green | 0.5% |
| Distilled water | 98.5% |

(12) Re-patch property to skin

The test piece which was cut into 15 mm x 70 mm was prepared. It was patched to the specified skin part (testing part), and immediately was re-patched to another surface of skin. The patch property of this case was scored according to the following criteria. The amount of corneum which was transferred to the adhesive surface of the patch material was large, the re-patch property was decreased, and the physically irritancy to the skin was increased.

3: possible to re-patch, 2: possible although the adhesion decreased a little, 3: no adhesion, and impossible

5. Inconspicuity of the patch material

In a thermostatic room under temperature 25°C and relative humidity 65%, the patch material which was cut into 15 mm x 50 mm was patched to a forearm inner front part of healthy 3 adult men in their 30-40s, and inconspicuity of each patch material was evaluated with appearance after 1 hour by the following 3 stages.

A: inconspicuous in 3 men
B: inconspicuous in 2 men
C: inconspicuous in 1 man

[Table 3]

| Entry | | Patch material | |
|---|---|---|---|
| | | Example 1 | Commercial Product a |
| Adhesive | | urethane | acryl |
| Adhesive layer (partly or whole) | | whole | partly (lattice pattern) |
| Water-vapor permeability of the patch material [g/m$^2$ ·day] | | 2,556 | 2,572 |
| Water-vapor permeability of the adhesive layer [g/m$^2$·day] | | 7,310 | - |
| | | | - |
| Adhesion to skin [N/15mm) | patching for 6 hours | 0.79 | 0.99 |
| | patching for 24 hours | 1.03 | 1.27 |
| Condition of adhesion (patching for 24 hours) | | ○ | ○ |
| Pain with peeling off (patching for 24 hours) | | ○ | ○ |
| Residue of adhesive (patching for 24 hours) | | ○ | Δ |

(continued)

| Entry | | Patch material | |
|---|---|---|---|
| | | Example 1 | Commercial Product a |
| Index of skin irritation | 1 hour after peeling off | 14 | 25 |
| | 24 hours after peeling off | 0 | 0 |
| Amount of peeled corneum [%] | patching for 24 hours | 25 | 80 |
| Re-patch property to skin | immediately after patching | ○ | Δ |
| Condition of adhesion: 4 or more to 5 or less of average of score is o, 2 or more to less than 4 is Δ, and 1 or more to less than 2 is ×. Pain with peeling off: 3 or more to 4 or less of average of score is o, 2 or more to less than 3 is Δ, and 1 or more to less than 2 is x. Residue of adhesive: 3 or more to 4 or less of average of score is o, 2 or more to less than 3 is Δ, and 1 or more to less than 2 is ×. Re-patch property to the skin: 2.5 or more to 3.0 or less of average of score is o, 2.0 or more to less than 2.5 is Δ, and 1.0 or more to less than 2.0 is ×. | | | |

The water-vapor permeability of the patch material of Example 1 was 2,556 g/m$^2$·day, while the water-vapor permeability of "Commercial Product a" was 2,572 g/m$^2$·day. "Commercial Product a" achieved the high water-vapor permeability by applying the adhesive in lattice pattern, whereas it was found that the patch material of Example 1 has very excellent water-vapor permeability even in the condition that the whole surface of the urethane resin film is covered by the adhesive layer. Also, for some adhesive tapes for the medical use which are commercially available now, the amount of peeled corneum was evaluated separately, and consequently all of them showed over 50%. In "Commercial Product a", the amount of peeled corneum of 80% was found in the part where the adhesive was applied, and the corneum was peeled off from almost the whole surface in the part where the adhesive was applied. In addition, the residue of adhesive was quite much relative to the patch material of Example 1, and there was the test subject who was found that the adhesive remained in the whole surface. On the other hand, the amount of peeled corneum of the patch material of Example 1 was 25%, which was significantly low, and there was no residue of adhesive, and it was found to be gentle-to-skin patch material.

Therefore, the patch material of the present invention has high moisture permeability and suitable adhesion, causes no pain, provides no residue of adhesive, has low irritancy to skin, provides low amount of peeled corneum, and has fine re-patch property.

[Table 4]

Table 4

| Entry | | Patch material | | | | | |
|---|---|---|---|---|---|---|---|
| | | Example 4 | Example 5 | Example 6 | Example 7 | Example 8 | Comparative Example 8 |
| Adhesive | | A | A | A | A | A | acryl adhesive |
| Thickness of the adhesive [µm] | | 5 | 10 | 15 | 20 | 25 | 5 |
| Property of the patch material | Total thickness [µm] | 10 | 17 | 22 | 27 | 32 | 10 |
| | Probe tack [N/5mmφ] | 0.27 | 0.30 | 0.38 | 0.41 | 0.50 | 0.31 |
| | Holding force [mm] | 0.30 | 0.20 | 0.10 | 0.10 | 0.10 | 0.10 |
| | Adhesion to Bakelite [N/15mm] | 0.96 | 0.92 | 0.96 | 1.09 | 1.22 | 0.94 |
| | Water-vapor permeability [g/m²·day] | 4065 | 3687 | 3349 | 3152 | 2998 | 2330 |
| Practical evaluation | Adhesion to skin [N/15mm] — patching for 24 hours | 0.48 | 0.66 | 0.76 | 0.84 | 0.87 | 0.62 |
| | Amount of peeled corneum [%] — patching for 24 hours | 8 | 12 | 14 | 16 | 17 | 64 |
| Inconspiscuity | | A | — | — | — | — | A |

Total thickness: an observed value of thickness of the obtained patch material was shown

Holding force: expressed in slid length after 30 minutes

Adhesion to Bakelite: measured in peeled angle 90°

In the Table 4, Examples 4-8 of the present invention showed comparable alternative characteristics and adhesion to skin, but 1/6 or less the amount of peeled corneum and about 1.7 times the water-vapor permeability, compared to Comparative Example 8. It was confirmed that Examples 4-8 of the present invention have good practical property.

**INDUSTRIAL APPLICABILITY**

The patch material of the present invention is applied to the skin or the like, for example, to be preferably used for a medical use, e.g. in the field such as medical and sanitary field, external use or the like. Concretely, it can be used in bandage, adhesive bandage, dressing material, surgical tape for a surgical operation, transdermal therapeutic drug or the like. The patch material of the present invention may take arbitrary forms e.g. film, sheet, plate, belt, tape or the like according to the embodiment. It may be stored in rolled form and used with cutting properly when it is used.

As one example, the present invention can provide the patch material which can withstand patching in specified time and has excellent moisture permeability, and causes low pain with peeling off, has low irritancy to the skin and provides low amount of peeled corneum.

**EXPRESSION OF SIGNS**

1    carrier
2    backing material
3    adhesive layer
4    release material

**Claims**

1. A patch material which comprises a backing material having a water-vapor permeability of 3,000 $g/m^2 \cdot day$ or more and an adhesive layer having a water-vapor permeability of 5,000 $g/m^2 \cdot day$ or more, wherein the adhesive layer comprises a polyurethane adhesive obtained by reacting

    (1) an active hydrogen compound having a number-average molecular weight of 5,000 or more and an average number of functional groups of 2 or more, and having a polyoxyalkylene structure,
    (2) an active hydrogen compound having a number-average molecular weight of 1,500-5,000 and an average number of functional groups of 1, and having a polyoxyalkylene structure, and
    (3) an organic polyisocyanate,

    wherein an average number of functional groups of all active hydrogen compounds used for obtaining the adhesive is 2-2.6, and a content of ethylene oxide units of all active hydrogen compounds in the adhesive is 3-8% by weight.

2. The patch material according to claim 1 wherein the active hydrogen compound of the (1) is polyether polyol having the average number of functional groups of 2-3.

3. The patch material according to claim 1 or 2 showing the water-vapor permeability of 2,000-4,000 $g/m^2 \cdot day$, wherein the adhesive layer essentially covers a whole surface of the backing material.

4. The patch material according to any one of claims 1 to 3 wherein the thickness of the adhesive layer is 10-40 $\mu m$.

5. The patch material according to any one of claims 1 to 4 wherein the thickness of the backing material is 5-10 $\mu m$, and the thickness of the adhesive layer is 3-7 $\mu m$.

6. The patch material according to any one of claims 1 to 5 wherein the backing material is Poly (ether urethane) resin film or Poly (ester urethane) resin film.

7. The patch material according to any one of claims 1 to 6 wherein the carrier is provided in a releasable form on a surface of the backing material on the side opposite to a surface on which the adhesive layer is provided.

8. The patch material according to any one of claims 1 to 7 wherein an average of adhesion to human skin of 10 people is approximately 0.7-1.2 N/15mm after 24 hours of patching.

9. The patch material according to any one of claims 1 to 8 for a medical use.

10. The patch material according to claim 9 wherein the patch material is a dressing material.

11. A polyurethane adhesive having a water-vapor permeability of 5,000 g/m$^2$·day or more, which is obtained by reacting

(1) an active hydrogen compound having a number-average molecular weight of 5,000 or more and an average number of functional groups of 2 or more, and having a polyoxyalkylene structure,
(2) an active hydrogen compound having a number-average molecular weight of 1,500-5,000 and an average number of functional groups of 1, and having a polyoxyalkylene structure, and
(3) an organic polyisocyanate,

wherein an average number of functional groups of all active hydrogen compounds used for obtaining the adhesive is 2-2.6, and a content of ethylene oxide units of all active hydrogen compounds in the adhesive is 3-8% by weight.

12. The urethane adhesive according to claim 11 wherein the content of ethylene oxide units of all active hydrogen compounds in the adhesive is 4-6% by weight.

**Patentansprüche**

1. Pflastermaterial, das ein Trägermaterial mit einer Wasserdampfpermeabilität von 3.000 g/m$^2$·Tag oder mehr und eine Haftmittelschicht mit einer Wasserdampfpermeabilität von 5.000 g/m$^2$·Tag oder mehr umfasst, wobei die Haftmittelschicht ein Polyurethanhaftmittel, erhalten durch Umsetzen

(1) einer aktiver Wasserstoff-Verbindung, die ein Zahlenmittel des Molekulargewichts von 5.000 oder mehr und eine Durchschnittsanzahl von funktionellen Gruppen von 2 oder mehr aufweist und eine Polyoxyalkylenstruktur aufweist,
(2) einer aktiver Wasserstoff-Verbindung, die ein Zahlenmittel des Molekulargewichts von 1.500-5.000 und eine Durchschnittsanzahl von funktionellen Gruppen von 1 aufweist und eine Polyoxyalkylenstruktur aufweist, und
(3) eines organischen Polyisocyanats,

umfasst,
wobei eine Durchschnittsanzahl von funktionellen Gruppen aller aktiver Wasserstoff-Verbindungen, verwendet zum Erhalten des Haftmittels, 2-2,6 beträgt und ein Gehalt an Ethylenoxideinheiten aller aktiver Wasserstoff-Verbindungen in dem Haftmittel 3-8 Gew.-% beträgt.

2. Pflastermaterial nach Anspruch 1, wobei die aktiver Wasserstoff-Verbindung von (1) Polyetherpolyol ist, das die Durchschnittsanzahl an funktionellen Gruppen von 2-3 aufweist.

3. Pflastermaterial nach Anspruch 1 oder 2, das die Wasserdampfpermeabilität von 2.000-4.000 g/m$^2$·Tag zeigt, wobei die Haftmittelschicht im Wesentlichen eine vollständige Oberfläche des Trägermaterials bedeckt.

4. Pflastermaterial nach einem der Ansprüche 1 bis 3, wobei die Dicke der Haftmittelschicht 10-40 $\mu$m beträgt.

5. Pflastermaterial nach einem der Ansprüche 1 bis 4, wobei die Dicke des Trägermaterials 5-10 $\mu$m beträgt und die Dicke der Haftmittelschicht 3-7 $\mu$m beträgt.

6. Pflastermaterial nach einem der Ansprüche 1 bis 5, wobei das Trägermaterial ein Poly(etherurethan)harzfilm oder ein Poly(esterurethan)harzfilm ist.

7. Pflastermaterial nach einem der Ansprüche 1 bis 6, wobei der Träger in einer lösbaren Form auf einer Oberfläche des Trägermaterials auf der Seite gegenüber einer Oberfläche, auf welcher die Haftmittelschicht bereitgestellt ist, bereitgestellt ist.

8. Pflastermaterial nach einem der Ansprüche 1 bis 7, wobei eine durchschnittliche Haftung gegenüber menschlicher Haut von 10 Menschen ungefähr 0,7-1,2 N/15mm nach 24 Stunden des Verbindens beträgt.

9. Pflastermaterial nach einem der Ansprüche 1 bis 8 zur medizinischen Verwendung.

10. Pflastermaterial nach Anspruch 9, wobei das Pflastermaterial ein Verbandszeug ist.

11. Polyurethanhaftmittel, das eine Wasserdampfpermeabilität von 5.000 g/m²·Tag oder mehr aufweist, welches erhalten wird durch Umsetzen

> (1) einer aktiver Wasserstoff-Verbindung, die ein Zahlenmittel des Molekulargewichts von 5.000 oder mehr und eine Durchschnittsanzahl von funktionellen Gruppen von 2 oder mehr aufweist und eine Polyoxyalkylenstruktur aufweist,
> (2) einer aktiver Wasserstoff-Verbindung, die ein Zahlenmittel des Molekulargewichts von 1.500-5.000 und eine Durchschnittsanzahl von funktionellen Gruppen von 1 aufweist und eine Polyoxyalkylenstruktur aufweist, und
> (3) eines organischen Polyisocyanats,

wobei eine Durchschnittsanzahl von funktionellen Gruppen aller aktiver Wasserstoff-Verbindungen, verwendet zum Erhalten des Haftmittels, 2-2,6 beträgt und ein Gehalt an Ethylenoxideinheiten aller aktiver Wasserstoff-Verbindungen in dem Haftmittel 3-8 Gew.-% beträgt.

12. Urethanhaftmittel nach Anspruch 11, wobei der Gehalt an Ethylenoxideinheiten aller aktiver Wasserstoff-Verbindungen in dem Haftmittel 4-6 Gew.-% beträgt.

**Revendications**

1. Matériau de timbre adhésif qui comprend un matériau de renforcement ayant une perméabilité à la vapeur d'eau de 3 000 g/m²·jour ou plus et une couche adhésive ayant une perméabilité à la vapeur d'eau de 5 000 g/m²·jour ou plus, dans lequel la couche adhésive comprend un adhésif à base de polyuréthane obtenu par mise en réaction de

> (1) un composé hydrogène actif ayant une masse moléculaire moyenne en nombre de 5 000 ou plus et un nombre moyen de groupes fonctionnels de 2 ou plus, et ayant une structure polyoxyalkylène,
> (2) un composé hydrogène actif ayant une masse moléculaire moyenne en nombre de 1 500 à 5 000 et un nombre moyen de groupes fonctionnels de 1, et ayant une structure polyoxyalkylène, et
> (3) un polyisocyanate organique,

dans lequel un nombre moyen de groupes fonctionnels de tous les composés hydrogène actif utilisés pour obtenir l'adhésif est de 2 à 2,6, et une teneur en unités oxyde d'éthylène de tous les composés hydrogène actif dans l'adhésif est de 3 à 8 % en poids.

2. Matériau de timbre selon la revendication 1, dans lequel le composé hydrogène actif du (1) est un polyéther polyol ayant un nombre moyen de groupes fonctionnels de 2 à 3.

3. Matériau de timbre selon la revendication 1 ou la revendication 2, présentant une perméabilité à la vapeur d'eau de 2 000 à 4 000 g/m²·jour, dans lequel la couche adhésive couvre sensiblement une surface totale du matériau de renforcement.

4. Matériau de timbre selon l'une quelconque des revendications 1 à 3, dans lequel l'épaisseur de la couche adhésive est de 10 à 40 μm.

5. Matériau de timbre selon l'une quelconque des revendications 1 à 4, dans lequel l'épaisseur de la couche de renforcement est de 5 à 10 μm, et l'épaisseur de la couche adhésive est de 3 à 7 μm.

6. Matériau de timbre selon l'une quelconque des revendications 1 à 5, dans lequel le matériau de renforcement est un film en résine poly(éther uréthane) ou un film en résine poly(ester uréthane).

7. Matériau de timbre selon l'une quelconque des revendications 1 à 6, dans lequel le support est fourni sous une forme anti-adhésive sur une surface du matériau de renforcement sur le côté opposé à une surface sur laquelle la couche adhésive est fournie.

8. Matériau de timbre selon l'une quelconque des revendications 1 à 7, dans lequel une moyenne de l'adhésion à la peau humaine de 10 individus est d'approximativement 0,7 à 1,2 N/15 mm après 24 heures de port de timbre.

9. Matériau de timbre selon l'une quelconque des revendications 1 à 8, destiné à une utilisation médicale.

10. Matériau de timbre selon la revendication 9, dans lequel le matériau de timbre est un matériau de pansement.

11. Adhésif à base de polyuréthane ayant une perméabilité à la vapeur d'eau de 5 000 g/m$^2$·jour ou plus, qui est obtenu par mise en réaction de

(1) un composé hydrogène actif ayant une masse moléculaire moyenne en nombre de 5 000 ou plus et un nombre moyen de groupes fonctionnels de 2 ou plus, et ayant une structure polyoxyalkylène,
(2) un composé hydrogène actif ayant une masse moléculaire moyenne en nombre de 1 500 à 5 000 et un nombre moyen de groupes fonctionnels de 1, et ayant une structure polyoxyalkylène, et
(3) un polyisocyanate organique,

dans lequel le nombre moyen de groupes fonctionnels de tous les composés hydrogène actif utilisés pour obtenir l'adhésif est de 2 à 2,6, et une teneur en unités oxyde d'éthylène de tous les composés hydrogène actif dans l'adhésif est de 3 à 8 % en poids.

12. Adhésif à base d'uréthane selon la revendication 11, dans lequel la teneur en unités oxyde d'éthylène de tous les composés hydrogène actif dans l'adhésif est de 4 à 6 % en poids.

Fig.1

**REFERENCES CITED IN THE DESCRIPTION**

**Patent documents cited in the description**

- JP 2003190205 A
- JP 2005218496 A
- WO 0043433 A1
- US 5045601 A
- EP 0597636 A1
- JP HEI7231910 B
- JP 2005058288 A